Europäisches Patentamt

(19) European Patent Office

Office européen des brevets

(11) Numéro de publication: **0 213 023**
**B1**

(12) **FASCICULE DE BREVET EUROPEEN**

(45) Date de publication du fascicule du brevet: **14.03.90**  (51) Int. Cl.⁵: **C 12 P 7/06**

(21) Numéro de dépôt: **86401676.1**

(22) Date de dépôt: **28.07.86**

(54) Procédé de production d'éthanol et de divers sous-produits à partir de céréales.

(30) Priorité: **07.08.85 FR 8512094**

(43) Date de publication de la demande:
**04.03.87 Bulletin 87/10**

(45) Mention de la délivrance du brevet:
**14.03.90 Bulletin 90/11**

(84) Etats contractants désignés:
**DE GB IT LU NL**

(56) Documents cités:
**FR-A-2 442 887**
**GB-A-2 091 293**
**US-A-2 698 826**
**US-A-3 236 740**
**US-A-4 287 304**
**US-A-4 361 651**

(73) Titulaire: **SOCIETE DES BREVETS VALPI**
**19 Bis rue Alexandre Dumas**
**F-80045 AMIENS (FR)**

(72) Inventeur: **Monceaux, Philippe**
**1695 rue Victor Hugo**
**Le Francport F-60750 Choisy au Bac (FR)**
Inventeur: **Segard, Emile**
**33 rue Hurtebise**
**F-60200 Compiegne (FR)**

(74) Mandataire: **Hirsch, Marc-Roger**
**Cabinet Hirsch 34 rue de Bassano**
**F-75008 Paris (FR)**

Courier Press, Leamington Spa, England.

EP 0 213 023 B1

## Description

La présente invention a pour objet un procédé de production d'éthanol et de récupération du gluten à partir de céréales, tout particulièrement du blé.

On connaît divers procédés de production d'éthanol à partir de céréales. En fait, tous ces procédés:

soit, nécessitent des opérations de meunerie, c'est-à-dire de fractionnement des moutures de céréales pour séparer les germes et les sons de la farine obtenue, ce qui conduit à des pertes d'amidon et de gluten entraînés dans la fraction germes et sons et donc à un rendement en éthanol plus faible, ainsi qu'à une production faible de gluten;

soit, évitent ces opérations de meunerie avec séparation des sons mais impliquent, par contre, un broyage de l'ensemble sans récupération du gluten tout en conduisant cependant à une production convenable d'éthanol.

Ainsi, le brevet US 3 236 740 se rapporte à un procédé dans lequel le sédiment obtenu contient moins de 4 à 5% de protéines et nécessite une étape de récupération d'un peu de gluten, étape qui ne requiert pas le procédé de l'invention.

De même le brever US 2 698 826 a pour objet un procédé dans lequel une conversion suivie d'une séparation est effectuée pour éliminer les fibres avant fermentation alors que selon la présente invention, la séparation a lieu après la fermentation et non avant, ce qui permet la récupération d'un élément protéine, ce qui est impossible dans le procédé connu de ce brevet US 2 698 826.

La présente invention a pour objet un procédé perfectionné de traitement des céréales permettant d'obvier les inconvénients des procédés connus d'une part et surtout, d'autre part, d'obtenir du gluten, (produit noble dont le prix de vente est relativement élevé) et de l'éthanol (que l'on peut utiliser à de nombreuses fins et surtout à titre de carburant) ainsi que des produits enrichis en protéines (en des teneurs supérieures à 20% des matières sèches). Ce procédé permet un épuisement poussé de la charge organique des eaux de traitement et permet une consommation d'eau réduite grâce à des recyclages élevés et une réduction importante de la charge des rejets.

La présente invention a pour objet un procédé de production d'éthanol et de gluten à partir de céréales, tout particulièrement de blé, comportant les étapes suivantes:

a. broyage des céréales et trempage du broyat obtenu dans de l'eau, sous agitation à une température inférieure ou égale à 50°C, le rapport massique broyat total/eau présente étant de l'ordre de 0,8 à 2,5;

b. séparation mécanique du mélange obtenu dans l'étape a. pour produire un sédiment contenant la majeure partie des matières insolubles et une suspension, ladite séparation étant effectuée de telle sorte que la distribution pondérale obtenue soit formée de 40 à 65% de sédiment et de 60 à 35% de suspension, le sédiment contenant de 40 à 55% de matière sèche et la suspension contenant de 10 à 40% de matière sèche;

c. séparation ultérieure de la suspension ainsi produite dans l'étape b. pour isoler une fraction insoluble contenant plus de 50% des protéines initialement présentes et consistant en gluten de ladite céréale et une fraction surnageante, cette séparation étant une séparation mécanique effectuée à une température inférieure ou égale à 50°C, de telle sorte que ladite fraction surnageante contienne au minimum 90% de l'amidon résiduel présent dans la suspension finale.

d. mélange du sédiment obtenu dans l'étape b. avec ladite fraction surnageante de l'étape c. pour obtenir une suspension finale contenant au minimum 90% de l'amidon initial, le mélange formé étant agité et ajusté par addition d'eau à une concentration maximale de 25 à 45% de matières sèches;

e. hydrolyse enzymatique de l'amidon contenu dans cette suspension finale de l'étape d., cette hydrolyse consistant en deux hydrolyses enzymatiques successives;

f. fermentation de la suspension hydrolysée de l'étape e. effectuée à l'aide d'une souche de levure alcoolique, mise en oeuvre à une température comprise entre 20 et 45°C et obtenue par l'action d'une souche consistant en Saccharomyces Cereviscae, Saccharomyces Pombe ou analogue;

g. séparation de la suspension fermentée de l'étape f. en un surnageant éthanolique et un sédiment constituant un aliment protéine;

h. distillation du surnageant éthanolique pour récupérer l'éthanol.

Selon une forme d'exécution de l'invention la phase surnageante est soumise à une nouvelle étape de séparation complémentaire des levures entrainées et de recyclage éventuel vers l'étape de fermentation (f).

La première hydrolyse enzymatique est due à l'action d'amylase, à un pH de l'ordre de 5,5 à 6,5, une température comprise entre 80 et 100°C et une durée de 90 à 150 minutes, l'addition continue d'amylase thermostable, ainsi que de ses activaters tels les ions $Ca^{++}$, étant ajustée de telle sorte que l'amidon initial soit hydrolysé en oligosides solubles.

La deuxième hydrolyse enzymatique est due à l'action d'amyloglucosidase, à un pH de 4 à 5, une température de 50 à 70°C et une durée de 12 à 72 heures, l'addition continue d'amyloglucosidase étant ajustée pour transformer les oligosides en sucres simples.

Suivant un autre mode encore de réalisation de l'invention, le mélange résultant de la fermentation dans l'étape f. est séparé en deux phases, la phase surnageante éthanolique ou effluent éthanolique qui sera soumise à une distillation éthanolique, et la phase résiduelle ou sédiment protéine qui constitue un aliment protéine, ladite phase surnageante étant éventuellement soumise à une nouvelle étape de séparation complémentaire des levures entraînées et de recyclage éventuel vers l'étape de fermentation f., le sédiment pouvant cependant subir un lavage de récupération des résidus alcooliques qu'il contient.

L'invention a, en outre, pour objet une installation comprenant successivement:

a. une zone de broyage des céréales et de trempage du broyat obtenu.

b. une zone de séparation mécanique de la suspension produite dans la zone a.;

c. une zone de séparation de la suspension produite dans la zone b.;

d. une zone de mélange du sédiment obtenu dans la zone c.;

e. une zone d'hydrolyse de l'amidon contenue dans la suspension obtenue dans la zone d.;

f. une zone de fermentation de la suspension hydrolysée de l'étape e.;

g. une zone de séparation de la suspension fermentée produite dans la zone f. en un surnageant éthanolique et un sédiment constituant un aliment protéine;

h. une zone de distillation du surnageant éthanolique.

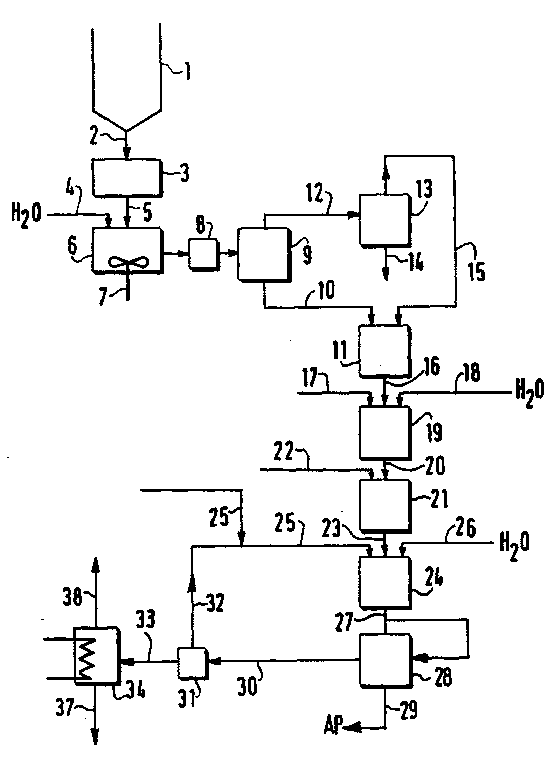

D'autres buts et avantages de l'invention apparaîtront à la lecture de la description suivante et de la figure donnée à titre non limitatif qui représente un schéma de principe du procédé selon l'invention.

Ce procédé décrit la transformation de blé en gluten de blé et en éthanol; l'invention ne saurait, en aucun cas, être limitée au traitement du blé et s'applique à toutes autres céréales. Par gluten, on entend non seulement gluten de blé, mais également le gluten de tout autre céréale telle orge, avoine, seigle, maïs, etc.

Dans l'installation représentée sur la figure jointe, la céréale, ici du blé, contenue dans le silo 1, est amenée par gravité par la conduite 2 dans le broyeur 3 où elle est broyée à une granulométrie inférieure à 4 mm. La masse broyée ou broyat est ensuite amenée par la conduite 5 dans l'enceinte de trempage 6 dans laquelle par la conduite 4 est amenée de l'eau. Cette enceinte de trempage contient des moyens d'agitation 7. Le trempage est effectué à la température de 30°C et l'eau est amenée selon un débit tel que le rapport massique broyat/eau soit de 1,0. La durée de trempage est en général inférieure à 2 heures. Après homogénéisation de la suspension dans un homogénéiseur, la suspension est envoyée dans un séparateur mécanique 9 consistant en un appareil de centrifugation ou d'hydrocyclonage. On retire du séparateur mécanique 9 par la conduite 10 un sédiment contenant 50% de matière sèche et formant 52% de la masse amenée dans le séparateur 9, ce sédiment étant conduit dans un mélangeur 11 dans lequel est amené, par la conduite 15, une fraction surnageante retirée d'un deuxième séparateur 13 dans lequel la suspension surnageante du séparateur mécanique 9 a été séparée en ladite fraction surnageante retirée par la conduite 15 et le gluten retiré du séparateur 13 par la conduite 14. La suspension surnageante retirée du séparateur mécanique 9 par la conduite 12 contient de l'ordre de 24% de matière sèche et forme 48% de la masse amenée par le séparateur 9. Le gluten ainsi produit peut subir tout traitement convenable, tel séchage pour en permettre la valorisation dans les conditions optimales.

Dans le mélangeur 11 se produit, à l'aide de moyens mécaniques non représentés, le mélange des fractions amenées par les conduites 10 et 15, lequel mélange est amené par la conduite 16 dans une première zone d'hydrolyse enzymatique 19 dans laquelle sont amenées, par la conduite 18, de l'eau, si nécessaire et, par la conduite 17, une amylase thermostable. Cette première hydrolyse est effectuée à un pH de 6,5 et à une température de 87°C pendant 120 minutes.

Pour activer l'amylase, 200 ppm d'un produit générateur d'ions $Ca^{++}$ est ajouté par la conduite 18. Il est important que, dans cette hydrolyse, les conditions opératoires soient adaptées pour que l'amidon présent soit hydrolysé en oligosides solubles.

La masse résultant de ce premier traitement d'hydrolyse est amenée par la conduite 20 dans une deuxième zone d'hydrolyse 21, permettant la transformation en sucres simples, dans laquelle est amenée par la conduite 22 une amyloglucosidase. Cette deuxième hydrolyse est effectuée à une température de l'ordre de 62°C et à un pH de l'ordre de 4,7 pendant 36 heures.

Les deux zones d'hydrolyse précitées peuvent, bien entendu, être remplacées par une zone unique de production de sucres simples ou être étagées en plusieurs zones successives opérant à des pH et températures étagées.

De la zone d'hydrolyse, la masse hydrolysée est amenée par la conduite 23 dans un fermenteur 24 alimenté en eau par la conduite 26 et ensemencé en levure consistant en Saccharomyces Cerevisiae, que l'on peut amener par une conduite d'alimentation 25. La température régnant dans le fermenteur 24 est de l'ordre de 30°C et la durée de séjour de la masse hydrolysée dans le fermenteur est de l'ordre de 5 heures. La masse fermentée est amenée par la conduite 27 dans un séparateur de phase 28 d'où l'on retire en cuve par la conduite 29 un aliment protéiné et par la conduite 30 une fraction éthanolique surnageante, cette fraction étant destinée, par distillation dans un appareil de distillation 34 à produire l'éthanol recherché. Il est possible préalablement à la distillation en 34 de soumettre ladite fraction éthanolique surnageante à une étape de séparation dans le séparateur 31 où sont récupérés les résidus de levure entraînés par la fraction résiduelle et non séparées dans le séparateur 28. Ces résidus de levure sont recyclés par la conduite 32 et la conduite d'alimentation 25 dans le fermenteur 24 tandis que la fraction éthanolique épurée est amenée par la conduite 33 dans l'appareil de distillation.

La fraction éthanolique quittant le séparateur de phase 28 est, soit amenée directement dans l'appareil de distillation 34 (mode de réalisation non représenté) ou, après passage dans le séparateur 31 (ainsi que représenté), cette fraction est soumise à une distillation dans l'appareil 34 et l'on retire l'éthanol produit par la conduite 38, tandis que les sous-produits contenus dans la fraction éthanolique brute amenée sont recueillies par la conduite 37 et, éventuellement, remélangés avec l'aliment protéiné obtenu en 29. L'aliment protéiné obtenu en 29 peut être séché, selon une méthode connue (par exemple sur cylindres) pour obtenir une farine à 10% d'humidité ou plus et contenant de l'ordre de 23% de protéines/matière sèche. Cet aliment peut être utilisé sous la forme de farine ou de pellets pour l'alimentation animale.

En fait, le bilan matière est représenté sur le tableau I suivant. Ile est exprimé pour 1000 kg de blé brut représentant 850 kg de matière sèche soit

N 5, 7: 100 kg

amidon: 560 kg

divers: 190 kg

Par N 5,7 on entend que la teneur en protéine est 5,7 fois la teneur en azote total.

eau = 1000 kg

gluten: 85 kg

-------amylase (0,5 kg/t d'amidon)

-------amyloglucosidase (1 l/t sucre soluble)

eau ———

1.600 kg

levures

—————————éthanol

350 l

aliment protéiné — 270 kg

## Revendications

1. Procédé de production d'éthanol et de gluten, à partir de céréales, tout particulièrement de blé, caractérisé en ce qu'il comporte les étapes suivantes:

a. broyage des céréales et trempage du broyat obtenu dans de l'eau, sous agitation à une température inférieure ou égale à 50°C, le rapport massique broyat total/eau présente étant de l'ordre de 0,8 à 2,5;

b. séparation mécanique du mélange obtenu dans l'étape a. pour produire un sédiment contenant la majeure partie des matières insolubles et une suspension, ladite séparation étant effectuée de telle sorte que la distribution pondérale obtenue soit formée de 40 à 65% de sédiment et de 60 à 35% de suspension, le sédiment contenant de 40 à 55% de matière sèche et la suspension contenant de 10 à 40% de matière sèche;

c. séparation ultérieure de la suspension ainsi produite dans l'étape b. pour isoler une fraction insoluble contenant plus de 50% des protéines initialement présentes et consistant en gluten de ladite céréale et une fraction surnageante, cette séparation étant une séparation mécanique effectuée à une température inférieure ou égale à 50°C, de telle sorte que ladite fraction surnageante contienne au minimum 90% de l'amidon résiduel présent dans la suspension finale;

d. mélange du sédiment obtenu dans l'étape b. avec ladite fraction surnageante de l'étape c. pour obtenir une suspension finale contenant au minimum 90% de l'amidon initial, le mélange formé étant agité et ajusté par addition d'eau à une concentration maximale de 25 à 45% de matières sèches;

e. hydrolyse enzymatique de l'amidon contenu dans cette suspension finale de l'étape d., cette hydrolyse consistant en deux hydrolyses enzymatiques successives;

f. fermentation de la suspension hydrolysée de l'étape e. effectuée à l'aide d'une souche de levure alcoolique, mise en oeuvre à une température comprise entre 20 et 45°C et obtenue par l'action d'une souche consistant en Saccharomyces Cereviscae, Saccharomyces Pombe ou analogue;

g. séparation de la suspension fermentée de l'étape f. en un surnageant éthanolique et un sédiment constituant un aliment protéiné;

h. distillation du surnageant éthanolique pour récupérer l'éthanol.

2. Procédé selon la revendication 1, caractérisé en ce que la phase surnageante est soumise à une nouvelle étape de séparation complémentaire des levures entrainées et de recyclage éventuel vers l'étape de fermentation (f).

3. Procédé selon la revendication 1 ou 2, caractérisé en ce que la première hydrolyse enzymatique est due à l'action d'amylase, à un pH de l'ordre de 5,5 à 6,5, une température comprise entre 80 et 100°C et une durée de 90 à 150 minutes, l'addition continue d'amylase thermostable, ainsi que de ses activateurs tels les ions $Ca^{++}$, étant ajustée de telle sorte que l'amidon initial soit hydrolysé en oligosides solubles.

4. Procédé selon la revendication 1 à 3, caractérisé en ce que la deuxième hydrolyse enzymatique est due à l'action d'amyloglucosidase, à un pH de 4 à 5, une température de 50 à 70°C et une durée de 12 à 48 heures, l'addition continue d'amyloglucosidase étant ajustée pour transformer les olygosides en sucres simples.

5. Installation pour la mise en oeuvre du procédé selon une quelconque des revendications 1 à 4, caractérisée en ce qu'elle comprend:

a. une zone (3) de broyage des céréales et de trempage (6) du broyat obtenu;

b. une zone (9) de séparation mécanique de la suspension produite dans la zone a.;

c. une zone (13) de séparation de la suspension produite dans la zone b.;

d. une zone (11) de mélange du sédiment obtenu dans la zone c.;

e. une zone (19, 21) d'hydrolyse de l'amidon contenue dans la suspension obtenue dans la zone d.;

f. une zone (24) de fermentation de la suspension hydrolysée de l'étape e.;

g. une zone (28) de séparation de la suspension fermentée produite dans la zone f. en un surnageant éthanolique et un sédiment constituant un aliment protéine;

h. une zone (34) de distillation du surnageant éthanolique.

## Patentanspruche

1. Verfahren zur Gewinnung von Äthanol und Gluten aus Getreide, insbesondere aus Weizen, das durch die folgenden Stufen gekennzeichnet ist:

a. Zermahlen des Getreides und Wässern des so erhaltenen Mahlguts, bei gleichzeitigem Rühren, wobei dieses Wässern bei einer Temperatur unterhalb oder gleich 50°C und mit einem Massenverhältnis gesamtes Mahlgut/benutzte Wassermenge zwischen 0,8 und 2,5 erfolgt;

b. mechanische Trennung der in Stufe a. erhaltenen Mischung zur Erzeugung eines Niederschlags, der den überwiegenden Teil der nichtlöslichen Stoffe enthält, und einer Suspension, wobei das durch die Trennung entstehende Massenverhältnis 40 bis 65% Niederschlag und 60 bis 35% Suspension ergibt, und der Niederschlag 40 bis 55% und die Suspension 10 bis 40% Trockensubstanz enthalten;

c. weitere Trennung der in Stufe b. erhaltenen Suspension zur Isolierung einer nichtlöslichen Fraktion, die mehr als 50% der ursprünglich enthaltenen Proteine enthält und aus Gluten des genannten Getreides besteht, und einer aufschwimmenden Fraktion, wobei diese Trennung eine mechanische Trennung ist, die bei einer Temperatur unterhalb oder gleich 50°C durchgeführt wird, und wobei diese aufschwimmende Fraktion mindestens 90% der in der Endsuspension entstehenden restlichen Stärke enthält;

d. Mischen des in Stufe b. enthaltenen Niederschlags mit aufschwimmender Fraktion der Stufe c. zur Erhaltung einer Endsuspension, die mindestens 90% der ursprünglichen Stärke enthält, wobei der erhaltenen Mischung bei gleichzeitigem Rühren soviel Wasser hinzugefügt wird, dass eine maximale Konzentration der Trockensubstanz von 25 bis 45% erreicht wird;

e. enzymatische Hydrolyse der in dieser Endsuspension in Stufe d. enthaltenen Stärke durch zwei aufeinanderfolgende enzymatische Hydrolysen;

f. Fermentierung der in Stufe e. hydrolysierten Suspension mit einem alkoholischen Hefestamm bei einer Temperatur zwischen 20 und 45°C, welcher Hefestamm aus Saccharomyces Cerevisiae, Saccharomyces Pombe oder ähnlichem gewonnen ist;

g. Trennung der in Phase f. fermentierten Suspension in eine ethanolische aufschwimmende Fraktion und einen Niederschlag, der ein proteinhaltiges Nahrungsmittel darstellt.

h. Distillierung der ethanolischen aufschwimmenden Fraktion zur Gewinnung von Äthanol.

2. Verfahren gemäss Anspruch 1, gekennzeichnet dadurch, dass die aufschwimmende Fraktion einer weiteren zusätzlichen Trennungsstufe der mitgeführten Hefen unterzogen wird und eventuell zur Fermentierungsstufe (f) rückgeführt wird.

3. Verfahren gemäss Anspruch 1 oder 2, gekennzeichnet dadurch, dass Amylase die erste enzymatische Hydrolyse bei einem pH-Wert zwischen 5,5 und 6,5, einer Temperatur zwischen 80 und 100°C und einer Dauer von 90 bis 150 Minuten bewirkt, wobei die thermostabile Amylase sowie deren Aktivatoren wie die $Ca^{++}$ Ionen fortlaufend so hinzugefügt werden, dass die ursprüngliche Stärke in lösliches Oligosid hydrolysiert wird.

4. Verfahren gemäss Anspruch 1 bis 3, gekennzeichnet dadurch, dass Amyloglucosidase die die zweite enzymatische Hydrolyse bei einem pH-Wert zwischen 4 und 5, einer Temperatur zwischen 50 und 70°C und einer Dauer von 12 bis 48 Stunden bewirkt, wobei Amyloglucosidase fortlaufend so hinzugefügt werden, dass die Oligoside in Einfachzucker umgewandelt werden.

5. Vorrichtung zur Durchführung des Verfahrens gemäss Anspruch 1 bis 4, gekennzeichnet dadurch, dass folgende Zonen bestehen.

a. Zone (3) zum Zermahlen des Getreides und zum Wässern des erhaltenen Mahlguts;

b. Zone (9) zur mechanischen Trennung der in Zone a. erhaltenen Suspension;

c. Zone (13) zur Trennung der in Zone b. erhaltenen Suspension;

d. Zone (11) zur Mischung des in Zone c. erhaltenen Niederschlags;

e. Zone (19, 21) zur Hydrolyse der Stärke, die in der in Zone d. erhaltenen Suspension enthalten ist;

f. Zone (24) zur Fermentierung der in Stufe e. hydrolisierten Suspension;

g. Zone (28) zur Trennung der in Zone f. gewonnenen Suspension in eine äthanolische aufschwimmende Fraktion und einen Niederschlag, der ein proteinhaltiges Nahrungsmittel darstellt;

h. Zone (34) zur Destillierung der äthanolischen aufschwimmenden Fraktion.

## Claims

1. Process for producing ethanol and gluten from cereals, more particularly wheat, characterized in that it comprises the following steps:

a. crushing of cereals and soaking the crushed cereals thus obtained in water, under stirring at a temperature lower than or equal to 50°C, the mass ratio of crushed material/water effectively present being comprised between 0.8 and 2.5;

b. mechanical separation of the mixture obtained in step a; so as to produce a sediment containing the major part of the insoluble substances and a suspension, the said separation being carried out in such a manner than the weight distribution obtained corresponds to 40 to 65% of sediment and 60 to 35% of suspension, said sediment containing 40 to 55% of dry substance and the suspension containing 10 to 40% of dry substance;

c. subsequent separation of the suspension thus obtained in step b. so as to isolate an insoluble fraction containing more than 50% of the proteins initially present and consisting of gluten of said cereal and a supernatent fraction, this separation being a mechanical separation carried out at a temperature lower than or equal to 50°C, so that the said supernatent fraction contains at least 90% of residual starch present in the final suspension;

d. mixture of the sediment obtained in step b. with said supernatent fraction of step c. to obtain a final suspension containing at least 90% of the initial starch, the mixture formed being stirred and adjusted by addition of water to a maximum concentration of 25 to 45% of dry substance;

e. enzymatic hydrolysis of the starch contained in this final suspension of step d., this hydrolysis consisting of two successive enzymatic hydrolyses;

f. fermentation of the hydrolysed suspension of step e. using a strain of alcoholic yeast, carried out at a temperature comprised between 20 and 45°C and obtained by the action of a strain consisting of Saccharomyces Cerevisiae, Saccharomyces Pombe or similar;

g. separation of the fermented suspension of step f. into a supernatent ethanol-containing fraction and a sediment consistuting a proteinized foodstuff;

h. distillation of said supernatent ethanol-containing fraction to recover the ethanol.

2. Process according to claim 1, characterized in that the supernatent phase is subjected to a supplementary step of separating any yeasts carried along and possible recycling towards the fermentation step (f).

3. Process according to claim 1 or 2, characterized in that the first enzymatic hydrolysis is due to the action of amyloglucinosidase, at pH in the range of 5.5—6.5, at a temperature comprised between 80 and 100°C and a duration of 90 to 150 minutes, the continuous addition of thermostable amylase, as well as its activators such as $Ca^{++}$ ions, being adjusted so that the initial starch is hydrolysed into soluble oligosides.

4. Process according to claims 1 to 3, characterized in that the second enzymatic hydrolysis is due to the action of amylogucosidase, at a pH of 4 to 5, a temperature of 50 to 70°C and a duration of 12 to 48 hours, the continuous addition of amyloglucosidase being adjusted in order to transfer the olygosides into simple sugars.

5. Installation for carrying out the process according to the any one of claims 1 to 4, characterized in that it comprises:

a. a zone (3) for crushing the cereals and soaking (6) said crushed cereals thus obtained;

b. a mechanical separation zone (9) for the suspension produced in zone a;

c. a separation zone (13) for the suspension produced in zone b.;

d. a zone (11) for mixing the sediment obtained in zone c.;

e. a zone (19, 21) for the hydrolysis of the starch contained in the suspension produced in zone d;

f. a zone (28) for the fermentation of the hydrolysed suspension of step e;

g. a zone (28) for the separation of the fermented suspension produced in the zone f. in a supernatent ethanol-containing fraction and a sediment constituting a proteinic foodstuff;

h. a zone (34) for the distillation of the supernatent ethanol-containing fraction.